# EUROPEAN PATENT SPECIFICATION

(11) **EP 3 632 313 B1**
(45) Date of publication and mention of the grant of the patent: **06.07.2022**
(21) Application number: 18805423.3
(22) Date of filing: 15.02.2018
(51) Int. Cl.: A61B 5/154

(54) **ADAPTER**
ADAPTER
ADAPTATEUR

(30) Priority: 26.05.2017 JP 2017104568
(43) Date of publication of application: 08.04.2020
(73) Proprietor: Kabushiki Kaisha Top, Adachi-ku, Tokyo 120-0035 (JP)
(72) Inventor: MANAKA, Yuki, Tokyo 120-0035 (JP)
(74) Representative: Schön, Christoph
(86) International application number: PCT/JP2018/005338
(87) International publication number: WO 2018/216272

(56) References cited:
- JP-A- 2003 245 267
- JP-A- 2012 066 090
- JP-U- S5 045 091
- JP-U- S61 123 216
- US-A1- 2012 150 129
- US-A1- 2015 201 877

## Description

### Technical Field

The present invention relates to an adapter.

### Background Art

Conventionally, a blood sampling kit has been used for collecting blood sample for a blood test and the like. The blood sampling kit collects blood from a human body by a blood sampler, and the collected blood sample is reserved in a vacuum blood collection tube attached to a blood sampling holder. Such blood sampling kit includes the blood sampler, an adapter to which the blood sampler is connected, the blood sampling holder to which the adapter is attached, and the vacuum blood collection tube. For the attachment of the adapter to the blood sampling holder, there are a type in which a male screw formed in the adapter is screwed to a female screw formed in the blood sampling holder and a type in which a locking member provided at the blood sampling holder is fit and engaged to a groove part formed in the adapter. An adapter applicable to both of the screw type and the engagement type has been proposed (for example, refer to Patent Document 1).

### Prior Art Documents

### Patent Literature

Patent Literature 1: Japanese Patent Application Laid-Open No. 2003-245267).

### Summary of Invention

### Technical Problem

In the conventional adapter, the male screw is extended from a substantially cylindrical body part in the base end direction, and an annular groove part is formed at a root of the male screw. As such, the adapter is attached to the blood sampling holder by screwing the male screw of the adapter to the female screw formed at the attachment part of the blood sampling holder in the case where the blood sampling holder is a screw type, while the adapter is attached to the blood sampling holder by engaging the locking member provided at the attachment part of the blood sampling holder to the annular groove part of the adapter in the case where the blood sampling holder is an engagement type.

Since the blood sampling needle is inserted in the human body, it is preferable that the blood sampling needle is maintained in a fixed state during the blood sampling. However, in the conventional adapter, there is a fear that the adapter might rotate with respect to the blood sampling holder since only the locking member of the blood sampling holder is engaged to the annular groove part.

In view of the above, it is an object of the present invention to provide an adapter capable of restricting the rotation of the adapter with respect to the engagement type blood sampling holder, even with an adapter corresponding to both types of blood sampling holder such as the screw type and the engagement type.

### Solution to Problem

[1] In order to achieve the above object, the adapter according to the present invention is an adapter attached to an attachment part in which a through-hole is formed and provided at a bottom part of a blood sampling holder which is a bottomed hollow cylinder,
wherein the adapter includes: a hollow body part which is connectable to a blood sampler for sampling blood from a human body and through which the sampled blood flows; a flange part which is provided at a base end side of the body part and which abuts the leading end surface of the attachment part; and a cylindrical protruding part which extends from the flange part in a direction opposite to the body part and which is inserted into the through-hole of the attachment part, and
a male screw part is formed in an outer periphery of the protruding part from the flange part side, and a cut-out part is formed in which a portion of the male screw part is cut out in a peripheral direction closer to a base end side than a leading end.

According to the present invention, the protruding part is inserted into the through-hole of the attachment part of the blood sampling holder, and the flange part is abut to the leading end surface of the attachment part. In the case of attaching the adapter to the screw type blood sampling holder, since the protruding part includes the male screw part, it is possible to screw the male screw part to the female screw part of the blood sampling holder.

Moreover, in the case of attaching the adapter to the engagement type blood sampling holder, since the male screw part is formed with a cut-out part closer to the base end side than the leading end of the male screw part, it is able to engage the protruding part by fitting the locking part urged by the biasing member to the cut-out part. Furthermore, since the cut-out part is formed at a part of the male screw part in the peripheral direction, when the locking member is fit to the cut-out part, the movement of the locking member is restricted by the thread ridge of the male screw part in the periphery of the cut-out part. Therefore, the rotation of the protruding part with respect to the attachment part is restrained.

As a result, even regarding the adapter corresponding to both types of blood sampling holders such as the screw type and the engagement type, it is able to restrict the rotation of the adapter in the case of using with the engagement type blood sampling holder,.

[2] Furthermore, according to the present invention, it is preferable that the cut-out part is a recessed part formed as a groove shape along an axis of the protruding part. With the recessed part having a groove shape along the axis of the protruding part, since the locking member is introduced into the recessed part, the interval between the locking member and the thread ridge of the male screw part in the periphery of the recessed part is decreased, thereby enabling to prevent the so-called wobble of the protruding part in the rotating direction and the adapter can be completely prevented from rotating.

### Brief Description of Drawings

FIG. 1 is an explanatory diagram illustrating a blood sampling kit of an embodiment of the present invention;
FIG. 2 is a side view of an adapter illustrated in FIG. 1;
FIG. 3 is a sectional view taken along line III-III of the adapter illustrated in FIG. 2;
FIG. 4 is an action diagram of attaching the adapter illustrated in FIG. 2 to a screw type blood sampling holder;
FIG. 5A is an explanatory diagram explaining a starting state of insertion of the adapter into an engagement type blood sampling holder;
FIG. 5B is an explanatory diagram explaining a state in which the adapter is attached to the engagement type blood sampling holder; and
FIG. 6 is a sectional view taken along line VI-VI of the blood sampling kit illustrated in FIG. 5B.

### Description of Embodiments

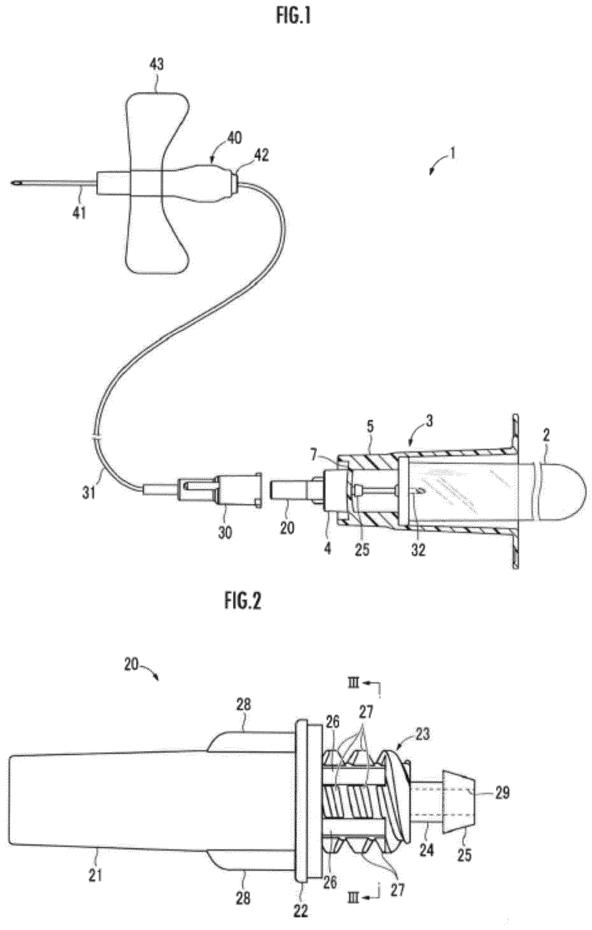

With reference to the drawings, a blood sampling kit 1 of an embodiment of the present invention is explained in details. As shown in FIG. 1, the blood sampling kit 1 collects blood from human body by a blood sampler 40 (hereinafter referred to as a butterfly needle), and the collected blood is reserved in a vacuum blood collection tube 2 attached to a blood sampling holder 3.

The blood sampling kit 1 includes a blood sampling holder 3 which is a bottomed hollow cylinder to which the vacuum blood collection tube 2 for storing the blood is attached, an adapter 20 attached to an attachment part 4 provided at a bottom part 7 of the blood sampling holder 3, a connector 30 connected to the adapter 20, a flexible tube 31 extending from the connector 30, and a butterfly needle 40 connected to the flexible tube 31.

The adapter 20 is provided with a needle tube 32 at the needle base 25. The needle tube 32 is covered by a rubber sleeve (not illustrated) for protecting the needle tube 32 until the adapter 20 is attached to the blood sampling holder 3. When attaching the adapter which is a separate body to the blood sampling holder 3, it is attached in a state in which the needle tube 32 is covered by the rubber sleeve. After the adapter 20 is attached to the blood sampling holder 3, the vacuum blood collection tube 2 is attached to the blood sampling holder 3 in a state of being pierced by the needle tube 32.

The butterfly needle 40 includes a hub 42 for supporting a needle tube 41 having a sharp front end and a pair of butterfly members 43 which are fit onto the hub 42.

Next, the adapter 20 is explained.

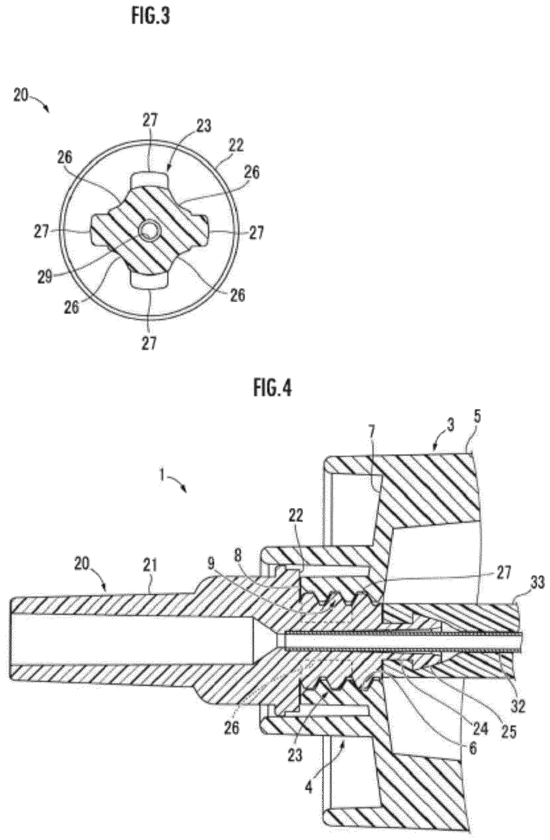

As shown in FIG. 2, the adapter 20 includes: a hollow body part 21 which is connectable to the butterfly needle 40 (refer to FIG. 1) via the connector 30 (refer to FIG. 1) and through which the sampled blood flows; a flange part 22 which is provided at a base end side of the body part 21 and which abuts the leading end surface 8 of the attachment part 4 (refer to FIG. 4); and a cylindrical protruding part 23 which extends from the flange part 22 in a direction opposite to the body part 21 and which is inserted into the through-hole (refer to FIG. 4) of the attachment part 4. The body part 21 is a lure tapered shape in which the connection opening is a round shape having a diameter of 4 mm and tapered by 6/100. In the present embodiment, although the body part 21 is a lure tapered shape, it may be a cylindrical shape or any tapered shape.

At the leading end side of the protruding part 23, a rubber sleeve fitting part 24 is formed to which the rubber sleeve is fitted, and at the leading end side of the rubber sleeve fitting part 24, a tapered needle base 25 is provided for guiding the protruding part 23 when inserting the protruding part 23 into the attachment part 4. A cut-out part 26 having a groove shape along the axis of the protruding part 23 is formed at the outer periphery of the protruding part 23.

In more details, a male screw part 27 is formed in the outer periphery of the protruding part 23, and a cut-out part 26 is formed by cutting out a portion of the male screw part 27 in a peripheral direction closer to the base end side (flange part side) than the leading end. Here, the cut-out part 26 may cut out not only less than a half but also half or more of the outer periphery of the protruding part 23 if there is a male screw part 27 in at least a part of the peripheral direction of the outer periphery of the protruding part 23. Moreover, the cut-out part 26 may have any shape as long as the locking member is able to fit and engage such as a cross-sectional V shape, a round hole to which the pin fits, or the like.

In the embodiment, the cut-out part 26 is a plurality of recessed parts forming a groove along the axis of the protruding part 23. Hereinafter, the cut-out part 26 will be referred to as the recessed part 26.

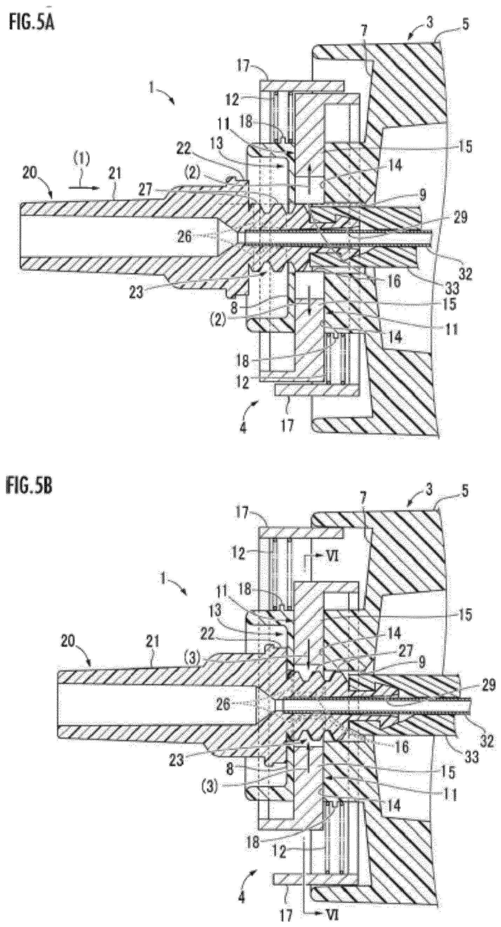

In the present embodiment, the recessed part 26 forming a groove along the axis is formed from the end portion of the flange part 22. However, it is not limited as such, and if the ridge of the male screw part 27 exists more closer to the needle base 25 than the recessed part 26, the recessed part 26 forming the groove along the axis may be formed on the way in the axis direction of the male screw part 27, since the locking member 11 which engages the recessed part 26 (refer to FIG. 5A and FIG. 5B) is restricted from moving in the axis direction by the ridge of the male screw part 27, thereby preventing the adapter 20 from coming off from the blood sampling holder 3.

Two ribs 28 are formed between the body part 21 and the flange part 22. Although two ribs 28 are formed in the present embodiment, it is not limited as such, and the ribs may be four or the like as long as the connector 30 can be guided when connecting the connector 30 to the adapter 20 (refer to FIG. 1). Furthermore, if the body part 21 and the flange part 22 have appropriate strength, the rib 28 may be omitted.

As shown in FIG. 3, the adapter 20 is formed with a through-hole 29 in which the needle tube 32 is inserted. The recessed part 26 forms a groove shape which is recessed so as to curve to the axis side. In the present embodiment, the recessed part 26 is formed to have a groove shape so as to curve to the axis side. However, it is not limited as such and the bottom of the recessed part 26 may be a plane surface as long as it is recessed with respect to the screw head of the male screw part 27 and the recessed part 26 can be engaged by the locking member 11 (refer to FIG. 5A and FIG. 5B).

The recessed part 26 is provided at positions equally dividing the outer periphery of the protruding part 23 into four. Although four recessed parts 26 are formed in the male screw part 27 in the present embodiment, the recessed part 26 may be plural such as two, or only one may be formed. Moreover, in the case of providing a plurality of recessed parts 26, they may be provided at unequal positions rather than positions which equally divide the outer periphery of the protruding part 23.

Next, a blood sampling holder 3 having a screw type attachment part 4, and the attachment of the adapter 20 to such blood sampling holder 3 will be explained.

As shown in FIG. 4, the blood sampling holder 3 includes: a holder body part 5 which is a bottomed hollow cylinder; and an attachment part 4 provided at a bottom part 7 of the holder body part 5 and formed with a through-hole 9. The attachment part 4 is formed with a female screw part 6 at a portion where the adapter 20 is inserted.

The protruding part 23 of the adapter is attached to the attachment part 4 by screwing the male screw part 27 of the adapter 20 to the female screw part 6, and abutting the flange part 22 to the leading end surface 8 of the attachment part 4. In the case of attaching the adapter 20 to the screw type attachment part 4, in order to obtain the necessary fitting strength or more, quite a number of screw threads will be necessary, requiring the length of the screw part of the male screw part 27 to have a certain length.

Here, in the adapter of the conventional art, an annular groove is formed from an end surface of the body part, and is formed so as to add the male screw from the leading end side of the annular groove. Therefore, the length from the end surface of the body part to the leading end of the male screw becomes long. Moreover, in the case where the female screw of the blood sampling holder side exists only at a position corresponding to the annular groove of the adapter, it is not able to completely fasten the male screw of the adapter, and the connecting is not possible.

Regarding this point, in the present invention, since the male screw part 27 is formed from the end surface of the flange part 22, it is possible to make the length from the end surface of the flange part 22 to the leading end of the male screw part 27 shorter compared to the conventional technology. Therefore, the adapter 20 of the present invention is able to be applied to both of the screw type and the engagement type blood sampling holders, and also able to shorten the length of the adapter 20. Moreover, since the male screw part 27 is formed from the end surface of the flange part 22, even if the female screw part 6 of the blood sampling holder 3 side is formed only at a position corresponding to the recessed part 26, the male screw part 27 can be completely fastened.

Next, the blood sampling holder 3 having the engagement type attachment part 4 is explained.

As shown in FIG. 5A, the blood sampling holder 3 includes a holder body part 5 which is a bottomed hollow cylinder and an attachment part 4 provided at a bottom part 7 of the holder body part 5. Hereinafter, the attachment part 4 is explained by a diagram expressed schematically.

The attachment part 4 includes: a sill 13 formed so as to protrude outward of the blood sampling holder 3 from the bottom part 7; a guide hole 14 formed in the sill 13 and which movably supports the locking members 11; a pair of locking members 11 which are inserted to the guide hole 14 and which are provided to be movable in a diameter direction and fit to the recessed part 26 to lock the protruding part 23; and a biasing member 12 which energizes each claw part 16 of the pair of locking members 11 to the axis side.

The locking member 11 includes: a locking body part 15 which is movably supported by the guide hole 14; the claw part 16 formed at the end of the locking body part 15 and fits the recessed part 26; a detouring member 17 which is extendedly provided so as to detour the sill 13 from the opposite side of the locking body part 15 from the claw part 16, and which is folded back in a U-shape in a side view.

The biasing member 12 is a compression spring supported by a spring seat 18 provided at the sill 13. By urging a part of the detouring member 17 positioned at the opposite side of the claw part 16 with respect to the axis of the protruding part 23, the claw part 16 is biased to the axis side.

Next, the attachment of the adapter 20 to the engagement type blood sampling holder 3 is explained.

A needle tube 32 is provided at the needle base 25 of the adapter 20, and the needle tube 32 is covered by a rubber sleeve 33. The protruding part 23 of the adapter 20 is inserted into the through-hole 9 of the attachment part 4 of the blood sampling holder 3 as indicated by arrow (1). The pair of locking members 11 are pushed open as indicated by arrows (2) by the rubber sleeve 33 and the male screw part 27 of the protruding part 23.

As shown in FIG. 5B, when the protruding part 23 of the adapter 20 is further inserted into the through-hole 9 of the attachment part 4, the flange part 22 abuts the leading end surface 8 of the attachment part 4. The claw part 16 of the locking member 11 which is biased to the axis side by the biasing member 12 fits the recessed part 26 and locks the protruding part 23. At the male screw part 27, since the ridge of the male screw part 27 is at the leading end side than the claw part 16 of the locking member 11, the movement of the claw part 16 of the locking member 11 to the axis direction is restricted serving as a stopper, thereby enabling to prevent the adapter 20 from falling out from the blood sampling holder 3.

In the blood sampling holder 3, the attachment part 4 includes: a locking member 11 which is provided movable in the diameter direction and fits the recessed part 26 (cut-out part) to lock the protruding part 23; and a biasing member 12 which urges the locking member 11 to the axis side. The locking member 11 urged by the biasing member 12 fits to the cut-out part 26 of the protruding part 23 inserted into the attachment part 4, and contacts the ridge of the male screw part 27, thereby locking the protruding part 23 and restricting the rotation of the protruding part 23.

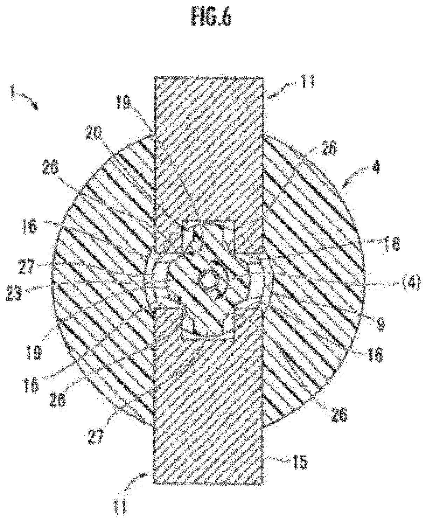

As shown in FIG. 6, since the recessed part 26 is formed at a part of the male screw part 27 in the peripheral direction, when the claw part 16 of the locking member 11 fits to the recessed part 26, the movement of the locking member 11 is relatively restricted by the ridge of the male screw part 27 in the circumference of the recessed part 26. Therefore, the rotation of the protruding part 23 with respect to the attachment part 4 is restrained.

Moreover, if the recessed part 26 is a groove along the axis of the protruding part 23, by guiding the claw part 16 of the locking member 11 by the recessed part 26, the gap between the ridge of the male screw part 27 in the circumference of the recessed part 26 and the claw part 16 of the locking member 11 becomes small, thereby eliminating the so-called rattling of the protruding part 23 in the rotating direction and preventing the adapter from rotating completely. Furthermore, at the back side in the figure of the recessed part 26 (the leading end side of the protruding part 23) and also the back side in the figure than the claw part 16, there is a leading end side of the male screw part 27 indicated by a hidden outline which serves as a stopper, thereby enabling to prevent the protruding part 23 from falling out from the attachment part 4.

Moreover, the recessed part 26 is recessed so as to smoothly curve to the axis side. On the other hand, in the embodiment, the part of the claw part 16 of the locking member 11 which abuts the recessed part 26 is formed as a corner part 19. However, this corner part 19 may be formed as a guiding curved surface part having a shape corresponding along the curved surface of the recessed part 26.

By forming the corner part 19 as the guiding curved surface part, when inserting the protruding part 23 of the adapter 20 into the attachment part 4, even if the claw part 16 does not fit to the recessed part 26 and rides onto the ridge of the male screw part 27, since the locking member 11 is biased to the axis side by the biasing member 12, the curved surface of the guiding curved surface part 19 slides the outer periphery of the ridge (crest of the thread)of the male screw part 27 to rotate the protruding part 23 as indicated by arrow (4), and the guiding curved surface part 19 fits to the recessed part 26. As such, by the guiding curved surface part 19, it is able to guide the recessed part 26 of the protruding part 23 to fit to the guiding curved surface part 19. As a result, it is able to restrict the rotation of the adapter 20 with respect to the blood sampling holder 3.

Moreover, in the embodiment, the locking member 11 is provided with one locking member body part 15 and two claw parts 16, and has a U-shape in planar view. By arranging two such locking members 11 having a U-shape in planar view as a pair of locking members 11, it is able to sandwich the protruding part 23 thereby enabling to lock four recessed parts 26.

As described above, the adapter 20 of the present invention can be applied to both of the screw type and engagement type blood sampling holders 3, and it is able to restrict the rotation of the adapter 20 also in the case of using the engagement type blood sampling holder 3.

Here, according to the present embodiment, the biasing member 12 and the locking member 11 are provided as separate elements, it is possible to integrally provide them by a resin component having elasticity. The engagement type attachment part 4 is not limited to the manner shown in FIG. 5A and FIG. 5B, and it may be any configuration as long as it can lock the cut-out part 26 (recessed part) of the adapter 20. Furthermore, in the present embodiment, although the blood sampler 40 is a butterfly needle, it is not limited as such, and it may be a single needle tube as long as it is capable of blood sampling.

### Description of Reference Numerals

3 ... blood sampling holder; 4 ... attachment part; 7 ... bottom part; 8 ... leading end surface; 9 ... through-hole; 20 ... adapter; 21 ... body part; 22 ...flange part; 23 ... protruding part; 26 ... cut-out part (recessed part); 27 ... male screw part; and 40 ... blood sampler (butterfly needle, needle tube).

## Claims

1. An adapter attached to an attachment part in which a through-hole is formed and provided at a bottom part of a blood sampling holder which is a bottomed hollow cylinder,
wherein the adapter comprises: a hollow body part which is connectable to a blood sampler for sampling blood from a human body and through which the sampled blood flows; a flange part which is provided at a base end side of the body part and which abuts the leading end surface of the attachment part; and a cylindrical protruding part which extends from the flange part in a direction opposite to the body part and which is inserted into the through-hole of the attachment part, and
at an outer periphery of the protruding part, a male screw part is formed from the flange part side, **characterized in that** a cut-out part is formed by cutting out a portion in a peripheral direction, closer to a base end side than a leading end, of the male screw part.

2. The adapter according to claim 1, wherein the cut-out part is a recessed part formed as a groove shape along an axis of the protruding part.

## Patentansprüche

1. Adapter, der an einem Befestigungsteil angebracht ist, in dem ein Durchgangsloch ausgebildet ist und das an einem unteren Teil eines Blutentnahmehalters, der ein Hohlzylinder mit Boden ist, vorgesehen ist,
wobei der Adapter folgendes umfasst: ein Hohlkörperteil, das mit einem Blutentnahmegerät zur Entnahme von Blut aus einem menschlichen Körper verbindbar ist und durch das das entnommene Blut fließt; ein Flanschteil, das an einer Basisendseite des Körperteils vorgesehen ist und das an die vordere Endoberfläche des Befestigungsteils anliegt; und ein zylindrisches vorstehendes Teil, das sich von dem Flanschteil in einer Richtung entgegengesetzt zu dem Körperteil erstreckt und das in das Durchgangsloch des Befestigungsteils eingesetzt ist,
wobei an einem Außenumfang des vorstehenden Teils ein Außengewindeteil von der Flanschteilseite ausgebildet ist, **dadurch gekennzeichnet, dass**
ein ausgeschnittenes Teil durch Ausschneiden eines Abschnitts in einer Umfangsrichtung näher zu einer Basisendseite als zu einem vorderen Ende des Außengewindeteils gebildet ist.

2. Adapter gemäß Anspruch 1, wobei das ausgeschnittene Teil ein vertieftes Teil ist, das als Nutform entlang einer Achse des vorstehenden Teils ausgebildet ist.

## Revendications

1. Adaptateur fixé à une partie de fixation dans laquelle un trou traversant est formé et qui est disposé au niveau d'une partie fond d'un porte-prélèvements de sang qui est un cylindre creux à fond,
dans lequel l'adaptateur comprend : une partie corps creux qui peut être reliée à un dispositif de prélèvement de sang pour prélever du sang d'un corps humain et à travers laquelle s'écoule le sang prélevé ; une partie bride qui est disposée au niveau d'un côté d'extrémité de base de la partie corps et qui vient en butée contre la surface d'extrémité avant de la partie de fixation ; et une partie saillante cylindrique qui s'étend à partir de la partie bride dans une direction opposée à la partie corps et qui est insérée dans le trou traversant de la partie de fixation, et
au niveau d'une périphérie externe de la partie saillante, une partie filetage mâle est formée à partir du côté de la partie bride, **caractérisé en ce que**
une partie découpée est formée par découpe d'une portion dans une direction périphérique, plus proche d'un côté d'extrémité de base que d'une extrémité avant, de la partie filetage mâle.

2. Adaptateur selon la revendication 1, dans lequel la partie découpée est une partie encastrée formée sous la forme d'un sillon le long d'un axe de la partie saillante.
